(19) Europäisches Patentamt
European Patent Office
Office européen des brevets

(11) Publication number: **0 405 583 A1**

(12) **EUROPEAN PATENT APPLICATION**

(21) Application number: **90112463.6**

(22) Date of filing: **29.06.90**

(51) Int. Cl.5: **C12M 1/40**, G01N 35/08, C12Q 1/40, C12Q 1/54, G01N 33/10

(30) Priority: **30.06.89 JP 170901/89**
**29.08.89 JP 222640/89**

(43) Date of publication of application:
**02.01.91 Bulletin 91/01**

(84) Designated Contracting States:
**DE GB**

(71) Applicant: **KANZAKI PAPER MANUFACTURING CO., LTD.**
**7, Kandaogawamachi 3-chome Chiyoda-ku**
**Tokyo 101(JP)**

(72) Inventor: **Hashizume, Yoshio**
**79-1 Nakatsu Kakogawa-cho**
**Kakogawa-shi Hyogo-ken 675(JP)**
Inventor: **Kariyone, Akio**
**Ota Manshon, 50-9 Ishii-cho**
**Nishinanajo Shimogyo-ku Kyoto 600(JP)**
Inventor: **Hayashi, Ryuzo**
**7-26 Hishiyanishi 5-chome**
**Higashiosaka-shi Osaka 577(JP)**
Inventor: **Oka, Minako**
**Gorindanchi H-2-63 5 Makomanai**
**Midorimachi 3-chome**
**Minami-ku Sapporo-shi Hokkaido 005(JP)**

(74) Representative: **Patentanwälte Beetz sen. - Beetz jun. Timpe - Siegfried - Schmitt-Fumian- Mayr**
**Steinsdorfstrasse 10**
**D-8000 München 22(DE)**

(54) **Method and apparatus for flow analysis with enzyme electrode.**

(57) The invention provides a device and a method for flow analysis which includes a mechanism for detecting a time period since the injection of a sample or a total volume of a buffer solution; and in which, the sample solution is fed at a flow rate set at the sample injection, and the flow of the sample solution is lowered or stopped and is increased or resumed after a certain time period. The method and device for flow analysis according to the invention maintains the advantage of the flow analysis, i.e., high-speed, and further attains the excellent sensitivity by making a sample or substrate remain near the surface of a working electrode (6) for a longer time.

*Fig.·1*

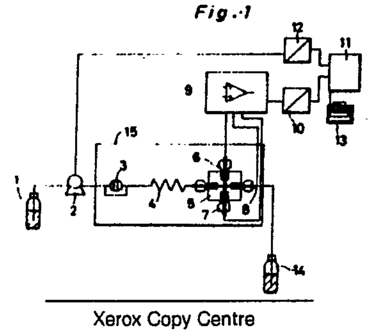

EP 0 405 583 A1

Xerox Copy Centre

# METHOD AND APPARATUS FOR FLOW ANALYSIS WITH ENZYME ELECTRODE

## BACKGROUND OF THE INVENTION

### 1. Field of the invention

The present invention relates to a technique and a apparatus for flow analysis using an enzyme-immobilized electrode (hereinafter referred to as an enzyme electrode), and more particularly to a method and a apparatus suitable for high-sensitive or multi-purpose analysis, e.g., for accurate and simple determination of the starch concentration in a sample.

### 2. Description of prior arts

Analyzers with an enzyme electrode are utilized in variety of fields, e.g., medical analysis, analysis of food, environmental analysis, because of its simplicity, speed and substrate-specificity. Especially flow analyzers with an enzyme working electrode equipped in a detection cell have advantages; high-speed analysis, simple operation, good reproducibility and easy automatization.

In analyzers with an enzyme electrode, it is not easy to change the detection range of concentration once the structure of the electrode, the type and the amount of the enzyme used are determined. Furthermore it is generally difficult for flow analyzers to detect a sample of a low concentration though that of a high concentration can be detected by dilution.

In flow analyzers with an enzyme electrode, sensitivity and accuracy of the determination depend upon both the diffusion and conversion rates of a substance in an immobilized enzyme membrane. Not like batch analysis, in flow analysis, a sample does not remain on the surface of the enzyme electrode but continuously flows. Hence, when enzyme reaction is sufficiently fast, sharp peaks are obtained; namely sensitivity and accuracy of the detection are ensured. Here fast enzyme reaction means either that the rate constant of the enzyme is relatively large or that an appropriate amount of the immobilized enzyme converts a substrate in a moment.

In slow enzyme reaction, before a substrate is diffused in the immobilized enzyme membrane and converted sufficiently, a high concentration region of the substrate passes through a detection cell and the substrate diffuses back from the immobilized enzyme membrane. Only part of the substrate is thus converted into a detectable form and sensitivity and accuracy of the detection decrease.

Recently mediator enzyme electrodes have been noted, in which an electron acceptor other than oxygen, e.g., ferrocene, ferricyanides and quinone coexists with an oxidoreductase such as oxidase and dehydrogenase. Electrons of the active site of oxidase, which is reduced with the oxidation of a substrate, are donated to the electron acceptor, and the concentration change of the electron acceptor is electrochemically detected. Oxidase can coexist with only a small amount of oxygen but can do with a large amount of a mediator. In the mediator enzyme electrodes with oxidase, thus, the saturation of response output due to deficiency of dissolved oxygen, which sometimes occurs in conventional enzyme electrodes with oxidase, is efficiently prevented. In the mediator enzyme electrodes with either oxidase or dehydrogenase, a potential applied to the electrode can be lowered, thereby diminishing the influence of a reducing substance in a sample. The mediator enzyme electrodes are, however, not preferably applicable to the flow analyzers. Because an oxidase-immobilized enzyme electrode using an mediator gives a slower response than that using oxygen as an electron acceptor.

In batch analyzers, since a sample remains in a detection cell, the enzyme reaction proceeds within the detection cell and a substance which is not directly detectable with an enzyme electrode, for example starch, can be analyzed. While conventional flow analyzers are not capable of analyzing such a substance.

Flow analyzers with an enzyme electrode for determining the concentration of starch naturally have similar problems.

Starch is industrially utilized as its solution or as processed or modified starch derivatives for food additives, adhesives, building materials, medicine and the like. It is, however, difficult to determine the concentration thereof accurately because it is giant molecules with a specific structure. Theoretically the accurate concentration of starch may be determined as follows: Starch is hydrolyzed into glucose: The concentration of glucose is then determined with an analyzer using an enzyme electrode. But the hydrolyzing process takes a relatively long time because starch consists of giant molecules. It is detrimental

to the advantage, i.e., high-speed analysis, of flow analyzers, and no effective methods have so far been proposed for that matter.

An objective of the invention is to provide a method and a apparatus with a simple mechanism suitable for high-sensitive or multi-purpose flow analysis.

Another object of the invention is to provide a method and a apparatus of flow analysis for determining the concentration of starch easily and accurately.

## SUMMARY OF THE INVENTION

The above and other related objectives of the invention are realized by a method of flow analysis, wherein a sample is injected into a buffer solution flow, and is introduced to an enzyme immobilized site which enzyme catalyzing a reaction of a substrate in the sample and also is introduced to a working electrode, and is then analyzed; and in which, when the sample is fed into or near the enzyme-immobilized site, a flow speed of the buffer solution is lowered or stopped and after a certain time period it is increased or resumed.

In one preferred embodiment of the method, the enzyme-immobilized site which enzyme catalyzing the reaction of the substrate in the sample and the working electrode are comprised in an enzyme-immobilized electrode; and in which, when the sample is fed into or near the enzyme-immobilized electrode, the flow speed of the sample solution is lowered or stopped and after the certain time period it is increased or resumed.

In another preferred embodiment of the method, for determining a concentration of starch, and the enzyme catalyzing the reaction of the substrate in the sample is an amylolytic enzyme or enzymes, and is filled in a column and the working electrode is an enzyme electrode having a membrane of immobilized glucose oxydase; and in which, when the sample is fed into or near the column, the flow speed of the sample solution is lowered or stopped and after the certain time period it is increased or resumed.

The objectives of the invention are also realized by a flow type analytical apparatus with an enzyme electrode comprising a pump for continuously feeding a buffer solution, a mechanism for injecting a sample into the buffer solution fed continuously, an enzyme-immobilized site which enzyme catalyzing a reaction of a substrate in the sample and a working electrode for detection; and further comprising a mechanism for detecting a time period since the injection of the sample or a total volume of the buffer solution , and a mechanism for lowering or stopping the flow of the sample solution and then for increasing or resuming it after a certain time period based on the time interval or the total volume of the solution since the sample is injected into the buffer solution to the sample is fed into or near the enzyme-immobilized site.

In one preferred embodiment of the apparatus includes the mechanism, for lowering or stopping the flow of the sample solution and for increasing or resuming it after a certain time period, includes a computer, a D/A converter and a pump.

Another preferred embodiment of the apparatus, the enzyme-immobilized site and the working electrode are comprised in an enzyme-immobilized electrode.

A further preferred embodiment of the apparatus further comprising a mechanism for determining the concentration of the substrate by detecting outputs from the enzyme electrode for the time period between the sample injection and conclusion of the measurement and by comparing a maximum variation of the outputs with a standard output without the sample.

In a still further preferred embodiment of the apparatus in which the enzyme-immobilized site which enzyme catalyzing the reaction of the substrate in the sample is an immobilized amylolytic enzyme or enzymes and filled in a column, and the working electrode is an enzyme electrode having a membrane of immobilized glucose oxydase for determining the concentration of starch.

Still another preferred embodiment of the apparatus, in which the amylolytic enzyme or enzymes are glucoamylase, $\alpha$-amylase or the both.

In still another preferred embodiment of the apparatus, mutarotase is immobilized at least either in the enzyme electrode or in the column for determining the concentration of starch.

An example of the mechanism utilizes a servo-motor driver controlled with an external voltage. In the mechanism, the speed of a motor is easily varied by connecting a D/A converter to an input/output circuit of a microcomputer (PIO) and by changing the set of the input/output circuit with a program. The time count or the measurement of the total volume of the buffer solution and the control of the pump may be performed with one microcomputer; then the smaller apparatus is provided.

An opening of an inlet at the sample injection is detected with a position detection sensor, a piezoelectric switch or the like. A time counting device incorporated in the microcomputer counts the time

period since the sample injection. The relationship between the flow and the pressure is previously determined with a pressure detection sensor inserted between the pump and the inlet. The pump is then controlled to make the pressure constant and the total volume of the buffer solution is thus accurately detected.

The timing for lowering or stopping the flow may be determined by calculation using the flow and the total volume between the inlet and the detection site. Alternatively, it may be determined as follows: Under the condition of a constant flow, the time period between the sample injection and the time at which a detectable peak is obtained is measured; Then, after the measured time period since the sample injection or a little earlier, the flow is lowered or stopped.

A further preferred embodiment of the apparatus includes a mechanism for determining the concentration of the substrate by detecting outputs from the enzyme electrode for a time period between the sample injection and conclusion of the measurement and by comparing the maximum variation of the outputs with a standard output without a sample.

In slow enzyme processes, a substrate is required to remain on the enzyme-immobilized site for a longer time. But the reduction of the flow rate causes an elongation of the time of analysis, and an advantage of the flow analytical apparatus, is lost. To attain the excellent sensitivity and the speed of analysis, the flow analytical apparatus of the invention includes a mechanism for detecting a time period since the injection of the sample or a total volume of the buffer solution, for lowering or stopping the flow of the sample solution after feeding the sample solution at a preset flow rate and then for increasing or resuming it after a certain time period.

The mechanism allows the substrate to remain in a required region, i.e., an enzyme-immobilized site, for a longer time and then to pass through the following region at the preset or even a greater flow rate, thus attaining the improved sensitivity without requiring a longer analytical time.

For example, when an enzyme electrode with oxidase immobilized thereon is used in a hydrogen peroxide electrode or oxygen electrode mode, the improved sensitivity is obtained by making a sample remain in the detection cell containing the enzyme electrode for a longer time.

The invention is also applicable to analysis with an mediator electrode.

For the detection of hydrogen peroxide, generally, the current caused by the following electrochemical reaction is measured with a working electrode, which is made by platinum or other conductive material.

$2H_2O_2 \rightarrow 2H_2O + O_2 + 2e^-$ (1)

Hydrogen peroxide is oxidized under the condition that the potential of the working electrode is maintained to be higher than +0.6V vs. a silver/silver chloride reference electrode. At this potential, a reducing substance, e.g., ascorbic acid, in a sample is also oxidized. Namely, the reducing substance interferes with the determination of hydrogen peroxide.

When a mediator, e.g., ferrocyanide ions, ferricyanide ions, phenazinemetosulfates, dichloroindophenol, benzoquinon, or anthraquinone derivatives, coexists in the buffer solution and peroxidase (hereinafter referred to as POD) exists in the buffer solution or on the surface of the electrode, the following reaction occurs.

$$H_2O_2 + 2[Fe(CN)_6]^{4-} + 2H^+ \xrightarrow{POD} 2[Fe(CN)_6]^{3-} + 2H_2O \qquad (2)$$

When ferricyanide ions formed in the reaction (2) are electrochemically reduced, the reduction current in proportion to the amount of hydrogen peroxide is observed. The electrochemical reaction (2) occurs under zero V potential against the silver/silver chloride reference electrode. At this potential of OV, the interfering substance, e.g., ascorbic acid, it not oxidized, that is, not interfere the determination of hydrogen peroxide.

However, reaction (2) goes slowly, and because ferrocyanide and ferricyanide ions permeable slowly into membrane of immobilized POD which is provided on the working electrode, reaction (2) goes even slower. Thus sufficient sensitivity is not obtained.

The use of free POD in the buffer solution increases the reaction speed, but this method has a disadvantage of free POD cannot be recovered and thereby the cost of analysis rises.

The method of the invention controls the time period for which a sample remains in the reaction site, POD immobilized site in this case, thus solving the problem.

The invention is also applicable to the following measurement, which has been possible only by batch analysis.

Namely, the invention makes enzymatic processes proceed within the detection cell and make analysis of a substance, which is generally not detectable only with an enzyme electrode, possible.

The invention is also applicable to the measurement; that is, to stop a sample before it enters the detection cell and to make it react for a certain time period. It is preferable that the system following the injection mechanism is set in a thermostate for stable enzyme reaction. For example, this method is applicable to determination of the concentration of starch. Glucoamylase is added to a sample containing starch or a slight amount of Glucoamylase is added into a buffer solution, and the sample is stopped before entering a detection cell. The enzymatic reaction, i.e., decomposition of starch into glucose, takes place before the detection cell. The concentration of glucose is then easily determined with an enzyme electrode having glucose oxidase immobilized thereon.

The apparatus of the invention preferably includes a mechanism for determining the concentration of a substrate by detecting outputs from the enzyme electrode for a time period between the sample injection and conclusion of the measurement and by comparing the maximum variation of the outputs with a standard output without a sample. Since no other data processing system is required in the above apparatus, it is especially advantageous when the apparatus is used for multi-purpose analysis.

For example, the concentration of an unknown substance is determined based on a calibration curve, which is obtained by comparing the peak of electric current with the minimum thereof, the value before a sample enter a detection cell.

In the another embodiment of the invention the concentration of starch is determined by providing an amylolytic enzymes-immobilized column and a working electrode.

In this embodiment, starch sample flowing in a buffer solution is completely decomposed into glucose by the immobilized amylolytic enzymes. And then the amount of glucose is measured and thereby the concentration of starch is determined.

By this method, the concentration of starch is accurately and easily determined irrespective of its type or molecular weight.

The amylolytic enzymes used in the invention may be any of those which effectively decompose starch into glucose. One or plural amylolytic enzymes are immobilized on carrier in a column. The preferable example is glucoamylase or α-amylase and the use of the both is more preferable.

Glucoamylase successively liberates glucose from the non-reducing terminal of starch molecules. α-amylase cuts starch molecules at random to liberate lower molecules. When glucoamylase and α-amylase coexist, decomposition of starch into glucose proceeds more efficiently; α-amylase cuts starch molecules into lower molecules and glucoamylase then successively cuts the lower molecules into glucose. The analytical speed is thus improved.

The same effect is obtained when puluranase or isoamylase coexists with glucoamylase.

Puluranase or isoamylase cuts α-1,6 bonds of starch molecules to lower molecules. But in practice, α-amylase has higher activity and longer life than the others.

In the apparatus of the invention, a column with an amylolytic enzyme or enzymes immobilized therein and an enzyme electrode with glucose oxidase immobilized thereon are provided. The amount of glucose formed in the column is measured with the enzyme electrode, and the concentration of starch is determined therefrom. In this method, the enzyme-immobilized site which enzyme catalying the reaction of the substrate in the sample is an immobilized amylolytic enzyme or enzymes and filled in a column, and the working electrode is an enzyme electrode having a membrane of immobilized glucose oxydase. As the reaction speed at the column with an amylolytic enzyme or enzymes is slower, it is preferred that the flow speed if the buffer solution is lowered or stopped when the sample is fed into or near the colum to get a superior effect.

Starch molecules are completely converted into glucose in the column and the glucose converted is then detected with the working electrode. In this method, glucose is used as standard in determination of starch concentration. Standard solutions of glucose are more easily prepared than those of starch because of its solubility.

α-D-glucose is liberated from starch molecules by the action of amylolytic enzymes while Glucoseoxidase acts on and oxidizes β-D-glucose.

α-D-glucose liberated from starch molecules in the column is spontaneously transformed into β-D-glucose with the elapse of sufficient time. In pure water it takes α-D-glucose more than several hours to coexist α-D-glucose and β-D-glucose in equilibrium state.

In a buffer solution containing phosphates and other components used in the measurement, the conversion of α-D-glucose into β-D-glucose is accelerated and the reaction reaches the equilibrium in only some minutes. Thus a sufficient time interval is preferably provided between the liberation of glucose from starch molecules by the action of amylolytic enzymes and the action of glucose oxidase onto glucose to

avoid the influence of the conversion.

When mutarotase exists in the column or the working electrode, the conversion into β-D-glucose proceeds more rapidly and the speed of analysis is improved. It is thus preferable that mutarotase is immobilized in the column or the working electrode.

Various types and shapes of enzyme-immobilized columns may be used, but tubing materials and the like used for flow injection analyzers, liquid chromotagraphs are preferable.

Covalent bonding method, physical adsorption method, encapsulation method and ion bonding method are known for immobilizing enzymes on a carrier.

In the encapsulation method, since starch, a measured substrate, is giant molecules, it does not easily diffuse in a carrier, thus not making contact with enzymes on the carrier. In the ion bonding method, the immobilization state varies depending on pH of a buffer solution and a sample. Thus the covering and ion bonding methods are not preferable in this invention.

In the physical adsorption method, enzymes are adsorbed in pores of a carrier. The carrier used in the method is, for example, sand, carbon, active carbon, silica gel, alumina, molecular sieve, titania, chitin, agarose gel, cellulose, tannin, silicon rubber, acid clay, diatomaceous earth and fire brick.

In the covalent bonding method, functional groups other than in the active site of enzyme protein or bonding site of a substrate are bonded with a carrier. For example, enzymes are immobilized on a carrier activated with cyanogen bromide or on a carrier having aromatic amino groups by ziazonium coupling. The carrier used in the method is, for example, cellulose, agarose, dextran, chitin, collagen, polyamino acid, polystyrene, polyacrylamid, polyvinyl alcohol, nylon, ion exchange resin, glass beads, zirconia, ceramics.

In the system that the flow rate is changed frequently like this invention, the covalent bonding method is preferable because it effectively prevents the leak of enzymes.

In the covalent bonding method, enzymes are cross-linked with a carrier by a reagent having not less than two functional groups. The cross linking agent is, for example, glutaraldehyde, toluene diisocyanate and hyxamethylene diisocyanate.

The immobilization method may be used alone or in combination. The carrier is preferably granular forms, and more preferably those of 10 to 1,000 µm in diameter.

The following methods are examples of immobilizing enzymes. Enzymes dissolved in a buffer solution are come into contact and combined with a carrier chemically treated and bonded to a cross linking agent on the surface thereof. Then a column is filled with the enzymes combined with the carrier. Alternatively, a column is filled with a carrier chemically treated and then immobilization of enzymes are carried out by feeding an enzyme solution.

The amount of enzymes immobilized on the carrier in the column depends on the type and shape of the carrier and immobilization ratio. The activation amount, sufficient to decompose starch of the upper limit concentration in measurement 100% in a time period in which starch stops or passes through at a low speed in the column, is immobilized. It is preferable that 1 to 1,000 times the activation amount, sufficient to decompose starch 100% in a time period in which starch stops or passes through at a low speed in the column, is immobilized.

When the flow of a starch solution is not stopped or reduced, a long column with a large amount of enzymes immobilized therein is required.

In this invention, the flow of a starch solution is temporarily stopped or reduced, a short column with a small amount of enzymes immobilized therein is sufficient. Furthermore, before and after the reaction in the column, the feed of the sample solution and detection are conducted at a higher speed, thus allowing the high-speed analysis.

In temporary stop of the flow, the preferable timing is when the whole sample enters the column. The stop time may be any as long as the high-speed analysis is attained. Practically stop within 5 minutes is preferable.

Temporary reduction of the flow speed is preferably conducted at least before the front of the sample emerges from the column. The preferable timing is when the front of the sample enter the column. The low speed is preferably maintained until the whole sample passes through and emerges the column. Reduction within 5 minutes is preferable.

Examples of the means for temporarily stopping or reducing the flow speed of the sample solution are as follows. The time period between the sample injection and time at which the sample solution reaches the column is determined by actual measurement or by calculation with the length and diameter of the tubing between the injection port and the column and the feeding speed of the pump. The feeding speed of the pump is changed based on the time period when the sample reaches the column.

The feeding speed of the pump is controlled via a D/A converter by a specific electronic circuit or a computer. The time period between a time of the sample injection and a time at which the sample reaches

the column may be accurately detected also by the electronic circuit or the computer.

There is a method that an enzyme electrode with amylolytic enzymes and glucose oxidase immobilized thereon is used and a sample solution is stopped near the enzyme electrode. In this method, the giant molecules of starch do not easily permeate through the enzymatic membrane immobilized on the electrode, and thereby the sensitivity is lowered and the time of analysis becomes longer. Thus this method is not preferable for the invention.

## BRIEF DESCRIPTION OF THE DRAWINGS

Other and further objects, features, and advantages of the invention will be more explicit from the following detailed description taken with reference to the drawings wherein:

Fig. 1 is a schematic view of a flow type analyzer according to the invention;

Fig. 2 shows results of measurement of Example 1 (o mark) and Reference 1 (● mark); and

Fig. 3 shows results of measurement of Example 2 (o mark), Example 3 (Δ mark) and Reference 2 (● mark).

## DETAILED DESCRIPTION OF EXAMPLES

Now referring to the drawings, examples of the invention are described below.

Hereinafter % means % by weight unless otherwise specified.

### Example 1

#### 1. Preparation of electrode

The side of a platinum wire of 2 mm in diameter is coated with a heat-shrinkable Teflon®, and one end of the wire is finished smoothly with a file and an emery paper of 1500 count. Electrolysis is conducted in 0.1 M sulfuric acid at +2.0V for five minutes with the platinum wire as the working electrode, a platinum plate of one centimeter squared as the counter electrode, and a saturated calomel electrode (hereinafter referred to as SCE) as the reference electrode. The platinum wire is washed well with water, is dried for 10 minutes at 40 °C, and is dipped for 1 hour in an anhydrous toluene solution containing 10% γ-aminopropyl triethoxy-silane, and is washed again. An enzyme is immobilized on the amino-silane treated platinum wire in the following manner.

5 mg of glucose oxidase (Sigma Co.; Type II) and 5 mg of bovine serum albumin (Sigma Co.; Fraction V) are dissolved in 1 ml of 100 mM sodium phosphate buffer solution (pH 7). Then glutaraldehyde is added to make the resulting concentration thereof 0.2% of the mixed solution. 5μl of the mixed solution is promptly placed on the prepared platinum wire, and is dried and cured at 40 °C for 15 minutes. The resulting electrode is stored in 100 mM sodium phosphate buffer solution (pH 6).

#### 2. Analyzer

The enzyme electrode prepared is incorporated as a working electrode 6 in a flow analyzer shown in Fig. 1. The analyzer includes an Ag/AgCl reference electrode 7 and a counter electrode of stainless steel pipe 8 connected to the outlet of a detection cell 5. These three electrodes are connected to a potentiostat 9. A voltage of + 0.6V against the Ag/AgCl reference electrode 7 is applied to the working electrode 6. Outputs from the potentiostat 9 are input into a microcomputer 11 via an A/D converter 10. The flow rate of a pump 2 is controlled by the microcomputer 11 via a D/A converter 12. Information relating to the control or alteration of the flow rate is previously stored in a RAM (read only memory) of the microcomputer 11 and is modified as occasion demands.

The line of the flow type analyzer following an injection port 3 is placed in a thermostat 15. The injection port 3 and the detection cell 5 are connected to each other via a mixing coil 4 composed of fluororesin tube (bore: 0.5mm; length: 1m). The measurement is conducted at 37 (±0.2 °C). The buffer solution used in measurement is 100 mM sodium phosphate solution (pH 6) containing 50 mM potassium

chloride and is stored in a buffer solution reservoir 1. Waste is discharged into a waste reservoir 14.

3. Method of analysis

The buffer solution is fed at the flow rate of 1 ml/min and 5 µl of 10 mM glucose aqueous solution is injected from the injection port 3, and the peak electric current and its position are read.

The instruction is then given to the microcomputer 11 for controlling the pump in the following manner. The sample solution is fed at 1 ml/min for a time period between the time at which a sample solution is injected and the time at which the peak top position is detected. The flow of the sample solution is stopped for 10 seconds at the time of the peak top position and is then resumed to 1 ml/min after a certain time period elapses. In this method, each measurement took 40 seconds.

Each 5 µl of 100 µM to 1 mM glucose aqueous solutions is injected from the injection port 3. A calibration curve (o mark) shown in Fig 2 is plotted by comparing the maximum value of the electric current obtained in measurement with the base value thereof. Although the concentration of glucose used in measurement is extremely low, the calibration curve shows excellent linearity.

Reference 1

1. Preparation of electrode

The electrode are prepared in the same manner as in Example 1.

2. Analyzer

The same analyzer as Example 1 is used except that the D/A converter 12 is removed here.

3. Method of analysis

Measured in the same manner as in Example 1 except that the flow rate is maintained to 1 ml/min. In this method, each measurement took 30 seconds.

The results are also shown in Fig. 2 (● mark). It is seen from Fig. 2 that Example 1 gives approximately 1.7 times greater sensitivity than Reference 1. The time period required for measurement is substantially the same in Example 1 and Reference 1, but the sensitivity with the same electrode is greatly improved.

Example 2

1. Preparation of electrode

The side of a platinum wire of 2 mm in diameter is coated with a heat-shrinkable Teflon®, and one end of the wire is finished smoothly with a file and an emery paper of 1500 count. Electrolysis is conducted in 0.1 M sulfuric acid at +2.0V for five minutes with the platinum wire as the working electrode, a platinum plate of one centimeter squared as the counter electrode, and an SCE as the reference electrode. The platinum wire is washed well with water, is dried for 10 minutes at 40 °C, is dipped for 1 hour in an anhydrous toluene solution containing 10% γ-aminopropyl triethoxy silane, and is washed again. An enzyme is immobilized on the amino-silane platinum wire in the following manner.

1 mg of peroxidase (Sigma Co.) and 9 mg of bovine serum albumin (Sigma Co.; Fraction V) are dissolved in 1 ml of 100 mM sodium phosphate buffer solution (pH 6). Then glutaraldehyde is added to make the resulting concentration thereof 0.5% of the mixed solution. 5 µl of the mixed solution is promptly placed on the prepared platinum wire, and is dried and cured at 40 °C for 15 minutes. The resulting electrode is stored in 100 mM sodium phosphate buffer solution (pH 6).

2. Analyzer

The same analyzer as Example 1 is used. The buffer solution used in measurement is 100 mM potassium phosphate solution (pH 6) containing 100 µM potassium ferrocyanide. A voltage of 0V against the Ag/AgCl reference electrode is applied to the working electrode. Since the reduction electric current is observed here, the symbol of the peak electric current reverses. Hereinafter the absolute value is used.

3. Method of analysis

The buffer solution is fed at the flow rate of 1 ml/min and 5 µl of 0.1 mM hydrogen peroxide aqueous solution is injected from the injection port 3, and the peak electric current and its position are read.

The instruction is then given to the microcomputer 11 for controlling the pump in the following manner. The sample solution is fed at 1 ml/min for a time between the time at which a sample solution is injected and the time at which the peak top position is detected. The flow of the sample solution is stopped for 5 minutes at the time of the peak top position and is then resumed to 1.2 ml/min. In this method, each measurement took 30 seconds.

Each 5 µl of 10 µM to 100 µM hydrogen peroxide aqueous solutions is injected from the injection port 3. A calibration curve (o mark) shown in Fig. 3 is plotted by comparing the maximum value of the electric current obtained in measurement with the base value thereof. Although the concentration of hydrogen peroxide used in measurement is relatively low, the sufficient calibration curve is obtained.

The calibration curve bends in the region of not less than 60 µM shown in Fig. 3. This is because ferrocyanides added to the buffer solution are oxidized and the concentration thereof on the surface of the enzyme electrode is lowered. Thus the speed of the enzymatic process is also decreased.

When the buffer solution does not contain ferrocyanides, the oxidation current of hydrogen peroxide is not observed and the electrode does not respond.

When 1 mM ascorbic acid aqueous solution is injected from the injection port 3, the interfering current is not observed.

Example 3

1. Preparation of electrode

The electrodes are prepared in the same manner as in Example 2.

2. Analyzer

The same analyzer as Example 2 is used

3. Method of analysis

The buffer solution is fed at the flow rate of 1 ml/min and 5 µl of 0.1 mM hydrogen peroxide aqueous solution is injected from the injection port 3, and the peak electric current and its position are read.

The instruction is then given to the microcomputer 11 for controlling the pump in the following manner. The sample solution is fed at 1 ml/min for a time period between the time at which a sample solution is injected and the time point at which the peak top position is detected. The flow of the sample solution is then reduced to 0.6 ml/min for 10 seconds at the time point of the peak top position and is then increased to 1.2 ml/min. In this method, each measurement took 25 seconds.

Each 5 µl of 10 µM to 100 µM hydrogen peroxide aqueous solutions is injected from the injection port 3. A calibration curve (Δ mark) shown in Fig. 3 is plotted by comparing the maximum value of the electric current obtained in measurement with the base value thereof. Although the concentration of hydrogen peroxide used in measurement is relatively low, the sufficient curve is obtained.

The saturation is not observed in the calibration curve of Example 3 shown in Fig. 3 since the value of the electric current is lower than Example 2.

Reference 2

1. Preparation of electrode

The electrodes are prepared in the same manner as in Example 2.

2. Analyzer

The same analyzer as Example 2 is used except that the D/A converter 12 is removed here.

3. Method of analysis

Measured in the same manner as in Example 1 except that the flow rate is maintained to 1 ml/min. In this method,each measurement took 35 seconds.

The results are also shown in Fig. 3 (● mark). It is seen from Fig. 3 that Example 2 gives approximately 4 times greater sensitivity than Reference 2, and Example 3 gives approximately 2 times greater sensitivity than Reference 2.

Example 4

1. Preparation of electrode

5 µl of 100 mM sodium phosphate buffer solution (pH 7), containing 3.0 mg/ml glucose oxidase (Sigma Co.; Type VII-S) and 7.0 mg/ml of bovine serum albumin (Sigma Co.; Fraction V) and 0.5% glutaraldehyde, is dropped onto a sufficiently cleaned platinum electrode of 2 mm in diameter and is treated at 40° C for 30 minutes. The enzyme-immobilized electrode is thus prepared.

2. Preparation of column

150 µl of an aqueous solution containing 0.5% glutaraldehyde is added to 150 mg of diatomaceous earth (diameter: 250 to 125 µm) treated by silane-coupling agent, and is treated at room temperature for 30 minutes. 200 µl of α-amylase (Sigma Co; Type XII-A) diluted to half with 100 mM sodium phosphate buffer solution (pH 7.0) is then added to the mixture and is immobilized.

150 µl of an aqueous solution containing 0.5% glutaraldehyde is added to 150 mg of diatomaceous earth treated by silane-coupling agent, and is treated at room temperature for 30 minutes. 100 µl of glucoamylase (Sigma Co; Aspergillus niger originated) diluted to a half with 100 mM sodium phosphate buffer solution (pH 7.0) is then added to the mixture and is immobilized.

The two types of enzyme-immobilized diatomaceous earth prepared are mixed and left at room temperature for 15 minutes. A Teflon®tube (outer diameter: 3mm; inner diameter: 2mm; length: 10cm) is filled with the mixed diatomaceous earth.

3. Analyzer

A flow analyzer shown in Fig. 1 is used for the measurement. The analyzer includes an injection port 3 (Rheodyne Co; Injector type 7125) for high performance liquid chromatography and a detection cell 5. The detection cell 5 has a working electrode 6 prepared in the above manner, an Ag/AgCl reference electrode 7 and a counter electrode of stainless steel pipe 8. Teflon®tubing (bore: 0.5 mm; length: 0.5 m) connects the injection port 3 to a mixing coil 4 working as an enzyme-immobilized column and the mixing coil 4 to the detection cell 5. The volume of the detection cell 5 is 40 µl. The working electrode 6 and the Ag/AgCl electrode 7 are disposed opposite to each other via the tubing for the buffer solution. A voltage of + 0.6V against the Ag/AgCl reference electrode 7 is applied to the working electrode 6 with a potentiostat 9. They

are placed in a thermostat 15. The temperature of the thermostat 15 is maintained 37 ± 0.2° C. The buffer solution is fed from a buffer solution reservoir 1 at 1.0 ml/min with a feed pump 2 for high performance liquid chromatography. The buffer solution used in measurement is 100 mM sodium phosphate solution containing 1 mM sodium azide.

The buffer solution after measurement is collected into a waste reservoir 14. Measurement values are recorded by a microcomputer 11.

4. Measurement of sample and results

The concentrations of 0.1% soluble starch and corn starch heated and gelificated were measured with the analyzer above. In measuring the concentration, the flow was stopped for approximately 100 seconds since the front of a sample reached the column. The flow rate before and after the stop was 1.0 ml/min. The results were expressed using the following equation.

(Value detected / starch concentration determined by glucoamylase decomposition method) x 100
Starch is decomposed 100% into glucose by a conventional glucoamylase decomposition method.
Both soluble starch and corn starch showed 100% decomposition.

Reference 3

1. Preparation of electrode

The electrodes are prepared in the same manner as in Example 4.

2. Preparation of column

Prepared in the same manner as Example 4.

3. Analyzer

The same analyzer as Example 4 is used.

4. Measurement of sample and results

The starch concentrations of the samples measured in Example 4 were measured at a constant flow rate 1.0 ml/min without stopping the flow of the sample solution. The results were compared with 100% decomposition value by the Glucoamylase decomposition method. Corn starch showed 52% decomposition and soluble starch 83%.

Example 5

1. Preparation of electrode

The electrodes are prepared in the same manner as in Example 4.

2. Preparation of column

50 µl of an aqueous solution containing 0.5% glutaraldehyde is added to 50 mg of diatomaceous earth (diameter: 250 to 125 µm) treated by silane-coupling agent, and is treated at room temperature for 30 minutes. 100 µl of mutarotase (Sigma Co; PORCINE KIDNEY originated) diluted to one tenth with 100 mM

sodium phosphate buffer solution (pH 7.0) is then added to the mixture and is immobilized.

250 µl of an aqueous solution containing 0.5% glutaraldehyde is added to 250 mg of diatomaceous earth treated by silane-coupling agent, and is treated at room temperature for 30 minutes. 100 µl of glucoamylase (Sigma Co; Aspergillus niger originated) diluted to a half with 100 mM sodium phosphate buffer solution (pH 7.0) is then added to the mixture and is immobilized.

The two types of enzyme-immobilized diatomaceous earth prepared are mixed and left at room temperature for 15 minutes. A Teflon® tube (outer diameter: 3 mm; inner diameter: 2mm; length: 10 cm) is filled with the mixed diatomaceous earth.

3. Analyzer

The same analyzer as Example 4 is used.

4. Measurement of sample and results

Measurement was performed in the same manner as Example 4. Both corn starch and soluble starch showed 100% decomposition.

Reference 4

1. Preparation of electrode

The electrode are prepared in the same manner as in Example 4.

2. Preparation of column

Prepared in the same manner as Example 5.

3. Analyzer

The same analyzer as Example 1 is used.

4. Measurement of sample and results

Measurement was performed in the same manner as Reference 3. Corn starch showed 40% and soluble starch 57%.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description and all changes which come within the meaning and the range of equivalency of the claims are therefore intended to be embraced therein.

## Claims

1. A method of flow analysis, wherein a sample is injected into a buffer solution flow, and is introduced to an enzyme-immobilized site which enzyme catalyzing a reaction of a substrate in the sample and also is introduced to a working electrode (6), and is then analyzed; and in which, when the sample is fed into or near the enzyme-immobilized site, a flow speed of the sample solution is lowered or stopped and after a certain time period it is increased or resumed.

2. The method of flow analysis as claimed in claim 1, in which the enzyme-immobilized site which enzyme

catalyzing the reaction of the substrate in the sample and the working electrode (6) are comprised in an enzyme-immobilized electrode; and in which, when the sample is fed into or near the enzyme-immobilized electrode, the flow speed of the sample solution is lowered or stopped and after the certain time period it is increased or resumed.

3. The method of flow analysis as claimed in claim 1, for determining a concentration of starch, and the enzyme catalyzing the reaction of the substrate in the sample is an amylolytic enzyme or enzymes, and is filled in a column and the working electrode (6) is an enzyme electrode having a membrane of immobilized glucose oxydase; and in which, when the sample is fed into or near the column, the flow speed of the sample solution is lowered or stopped and after the certain time period it is increased or resumed.

4. A flow type analytical apparatus with an enzyme electrode, comprising a pump (2) for continuously feeding a buffer solution, a mechanism (3) for injecting a sample into the buffer solution fed continuously, an enzyme-immobilized site which enzyme catalyzing a reaction of a substrate in the sample and a working electrode (6) for detection; and further comprising a mechanism for detecting a time period since the injection of the sample or a total volume of the buffer solution, and a mechanism for lowering or stopping the flow of the sample solution and then for increasing or resuming it after a certain time period based on the time interval or the total volume of the solution since the sample is injected into the buffer solution to the sample is fed into or near the enzyme-immobilized site.

5. The flow type analytical apparatus as claimed in claim 4, in which the mechanism, for lowering or stopping the flow of the sample solution and for increasing or resuming it after the certain time period, includes a computer (11), a D/A converter (12) and a pump (2).

6. The flow type analytical apparatus as claimed in claim 4 in which the enzyme-immobilized site and the working electrode (6) are comprised in an enzyme-immobilized electrode.

7. The flow type analytical apparatus as claimed in claim 6, further comprising a mechanism for determining the concentration of the substrate by detecting outputs from the enzyme electrode for the time period between the sample injection and conclusion of the measurement and by comparing a maximum variation of the outputs with a standard output without the sample.

8. The flow type analytical apparatus as claimed in claim 4, for determining the concentration of starch, in which the enzyme-immobilized site which enzyme catalyzing the reaction of the substrate in the sample is an immobilized amylolytic enzyme or enzymes and filled in a column, and the working electrode (6) is an enzyme electrode having a membrane of immobilized glucose oxydase.

9. The flow type analytical apparatus as claimed in claim 8, for determining the concentration of starch, in which the amylolytic enzyme or enzymes are glucoamylase, α-amylase or the both.

10. The flow type analytical apparatus as claimed in claim 8, for determining the concentration cf starch, in which mutarotase is immobilized at least either in the enzyme electrode or in the column.

Fig. 1

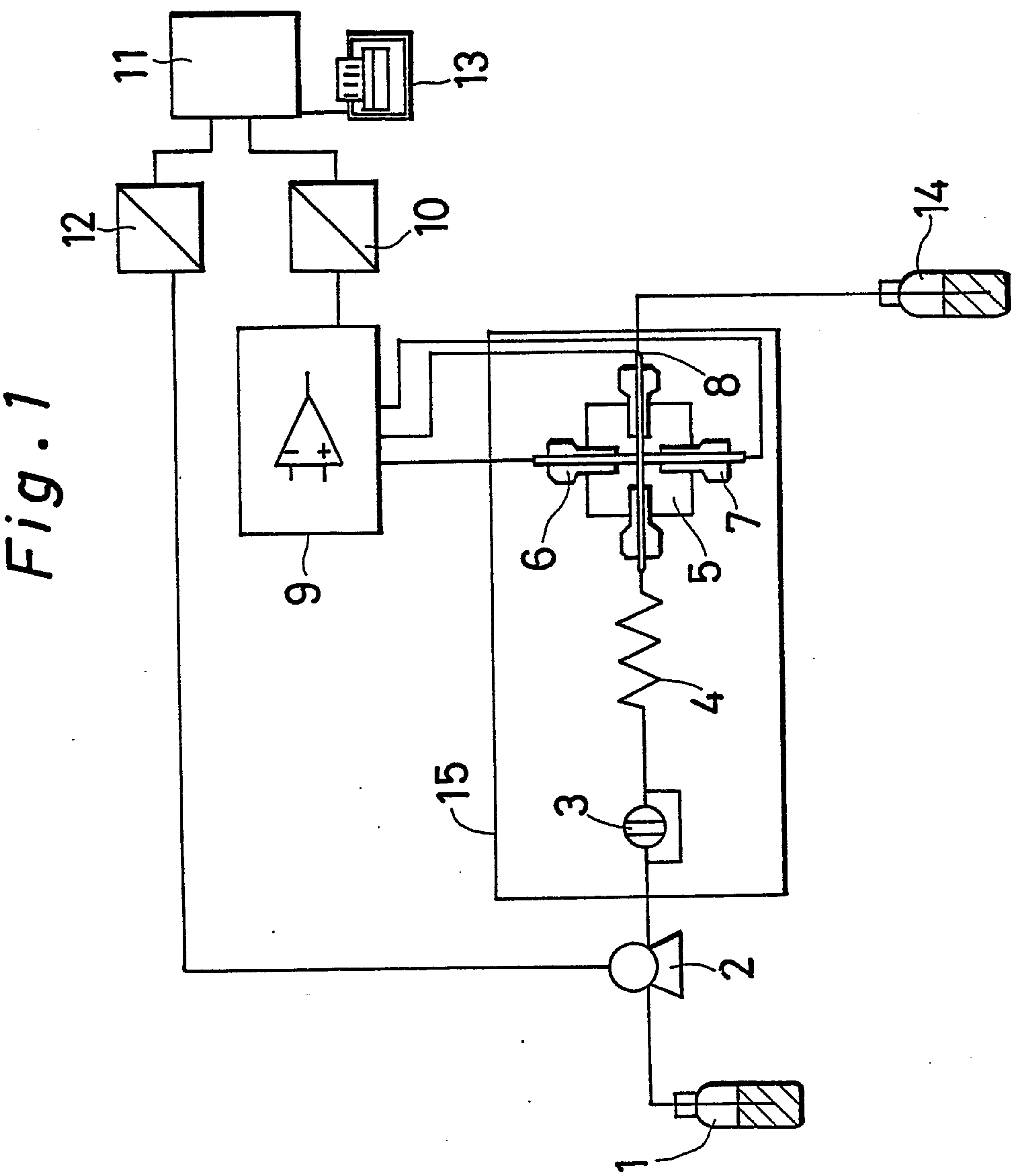

Fig. 2

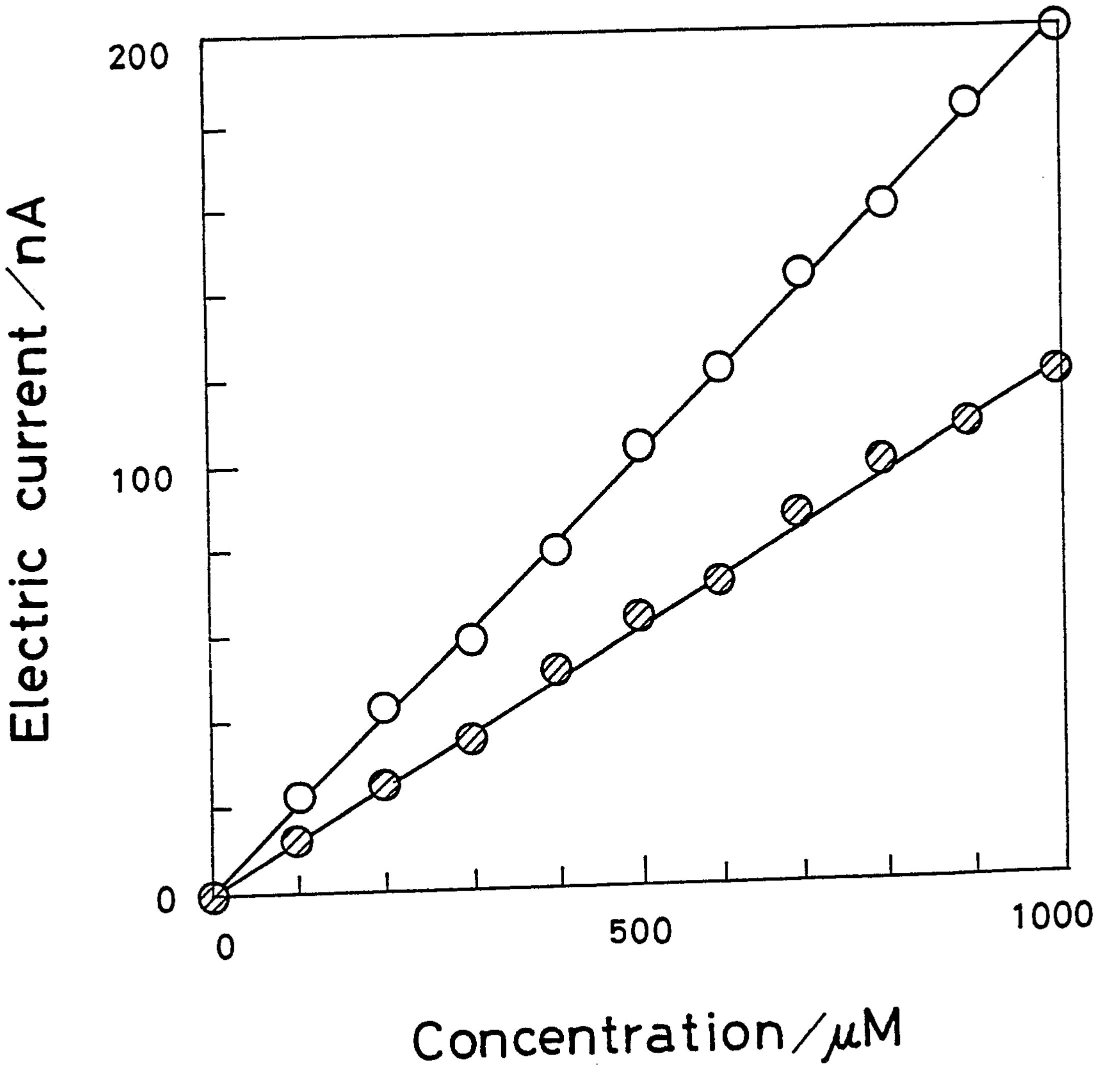

*Fig. 3*

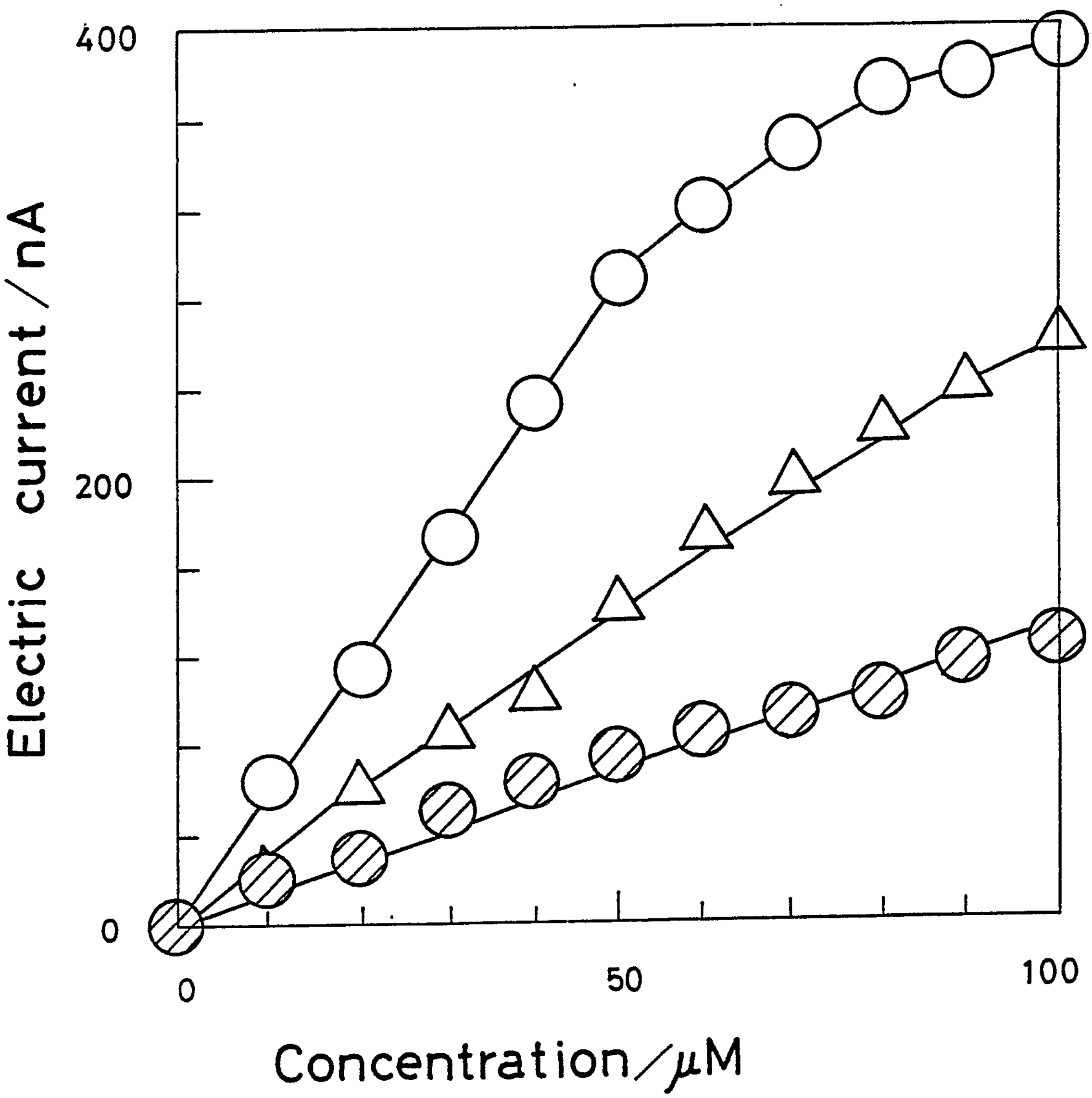

European Patent Office

# EUROPEAN SEARCH REPORT

Application Number

EP 90 11 2463

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,Y | CHEMICAL ABSTRACTS, vol. 112, 1990 Columbus, Ohio, USA Gorton et al.: "Determination of starch and maltose using immobilized amyloglucosidase and a glucose electrode in a flow injection system" page 389; right-hand column; ref. no. 3882F * abstract * | 1-10 | C12M1/40<br>G01N35/08<br>C12Q1/40<br>C12Q1/54<br>G01N33/10 |
| Y | Analytica Chimica Acta vol. 218, April 1989, Amsterdam, The Netherlands pages 291 - 301; Ruzicka et al.: "Characterization of immobilized enzymes by flow-injection techniques withvariable forward flow" * page 291, line 1 - page 294, line 35 * | 1-10 | |
| Y | CHEMICAL ABSTRACTS, vol. 109, 1988 Columbus, Ohio, USA Larew et al.: "Flow-injection determination of starch and total carbohydrate with an immobilized glucoamylase reactor and pulsed amperometric detection" page 880; left-hand column; ref. no. 1418147 * abstract * | 1-9 | TECHNICAL FIELDS SEARCHED (Int. Cl.5)<br>C12M<br>G01N<br>C12Q |
| Y | GB-A-2023286 (BIFOK AB) * page 1, line 41 - page 2, line 21; claims 1, 3, 7 * | 1-9 | |

The present search report has been drawn up for all claims

| Place of search | Date of completion of the search | Examiner |
|---|---|---|
| THE HAGUE | 18 OCTOBER 1990 | EPAILLARD P.J.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document



EPO FORM 1503 03.82 (P0401)